# EUROPÄISCHE PATENTSCHRIFT

(11) **EP 1 204 624 B1**
(45) Veröffentlichungstag und Bekanntmachung des Hinweises auf die Patenterteilung: **03.11.2004**
(21) Anmeldenummer: 00958472.3
(22) Anmeldetag: 18.08.2000
(51) Int. Cl.: C07C 29/16

(54) **KONTINUIERLICHES VERFAHREN ZUR HYDROFORMYLIERUNG VON OLEFINEN MIT 6 BIS 20 KOHLENSTOFFATOMEN**
CONTINUOUS PROCESS FOR HYDROFORMYLATING OLEFINS WITH 6 TO 20 CARBON ATOMS
PROCEDE CONTINU D'HYDROFORMULATION D'OLEFINES AYANT 6 A 20 ATOMES DE CARBONE

(30) Priorität: 20.08.1999 DE 19939491
(43) Veröffentlichungstag der Anmeldung: 15.05.2002
(73) Patentinhaber: BASF AKTIENGESELLSCHAFT, 67056 Ludwigshafen (DE)
(72) Erfinder: GRENACHER, Armin, Volker, D-67112 Mutterstadt (DE); STEPP, Hans, D-67161 Gönnheim (DE)
(74) Vertreter: Kinzebach, Werner, Dr.
(86) Internationale Anmeldenummer: PCT/EP2000/008095
(87) Internationale Veröffentlichungsnummer: WO 2001/014297

(56) Entgegenhaltungen:
- EP-A- 0 850 905
- DE-A- 2 139 630
- DE-A- 2 404 855

## Beschreibung

Die vorliegende Erfindung betrifft ein Verfahren zur Hydroformylierung von Olefinen mit 6 bis 20 Kohlenstoffatomen.

Die Hydroformylierung oder Oxo-Synthese ist ein wichtiges großtechnisches Verfahren und dient der Herstellung von Aldehyden aus Olefinen, Kohlenmonoxid und Wasserstoff. Diese Aldehyde können gegebenenfalls im gleichen Arbeitsgang oder nachfolgend in einem getrennten Hydrierschritt mit Wasserstoff zu den entsprechenden Alkoholen hydriert werden. Die Hydroformylierung erfolgt in Anwesenheit von homogen im Reaktionsmedium gelösten Katalysatoren. Als Katalysatoren werden dabei im Allgemeinen die Carbonylkomplexe von Metallen der VIII. Nebengruppe, insbesondere Co, Rh, Ir, Pd, Pt oder Ru eingesetzt, die unmodifiziert oder z. B. mit amin- oder phosphinhaitigen Liganden modifiziert sein können. Eine zusammenfassende Darstellung der großtechnisch ausgeübten Verfahren findet sich bei J. Falbe, "New Syntheses with Carbon Monoxide", Springer Verlag 1980, S. 162 ff.

Während kurzkettige Olefine mit bis zu 5 Kohlenstoffatomen gegenwärtig überwiegend mit ligandenmodifizierten Rhodiumcarbonylen als Katalysator hydroformyliert werden, behauptet bei den längerkettigen Olefinen Kobalt als katalytisch wirksames Zentralatom seine dominierende Stellung. Dies liegt zum einen an der hohen katalytischen Aktivität des Kobaltcarbonylkatalysators unabhängig von der Lage der olefinischen Doppelbindungen, der verzweigungsstruktur und der Reinheit des umzusetzenden Olefins. Zum anderen kann der Kobaltkatalysator von den Hydroformylierungsprodukten vergleichsweise leicht abgetrennt und in die Hydroformylierungsreaktion zurückgeführt werden. Außerdem können Katalysatorverluste bei der Aufarbeitung aufgrund des geringeren Preises von Kobalt leichter toleriert werden.

Den bekannten Verfahren zur Abtrennung und Rückführung des Kobaltkatalysators liegen im Wesentlichen zwei Prinzipien zugrunde: (1) der Kreislauf findet ohne Valenzänderung statt, d. h. das Kobalt verbleibt während des gesamten Zyklus von Hydroformylierung, Abtrennung und Rückführung in den Reaktor in der formal negativ einwertigen Form; und (2) während des Kreislaufs findet ein Valenzwechsel am Zentralatom vom negativ einwertigen zum metallischen bzw. posititv zweiwertigen Kobalt statt.

Der Prototyp des Prinzips (1) ist der sogenannte Kuhlmann-Prozess (vgl. US 3,188,351). Bei diesem Verfahren wird der homogen im Reaktionsaustrag gelöste Hydroformylierungskatalysator in Form von Kobaltcarbonylwasserstoff mit Hilfe von Sodalösung in sein wasserlösliches Natriumsalz überführt und in Wasser extrahiert. Nach der Phasentrennung wird aus der wässrigen alkalischen Lösung durch Umsetzen mit verdünnter Schwefelsäure der flüchtige Kobaltcarbonylwasserstoff wieder freigesetzt, mit Oxogas ausgestrippt, von dem umzusetzenden Olefin aufgenommen und in den Reaktor zurückgeführt. Dieses verfahrensprinzip erfordert jedoch wegen der Instabilität des Kobaltcarbonylwasserstoffs und der großen Zahl nacheinander zu vollziehender Schritte eine Reihe aufwendiger technischer Vorkehrungen.

Einfacher sind die auf dem Prinzip (2) beruhenden Verfahren, bei denen die organische Phase des Reaktoraustrags von den Kobaltcarbonylkomplexen durch Behandlung mit Sauerstoff oder Luft in Gegenwart von schwach saurem Wasser befreit wird (vgl.
DE-AS 2404855). Dabei wird der Kobaltkatalysator oxidativ zerstört und das Zentralatom formal von der Oxidationsstufe -1 nach +2 überführt und kann sodann durch Extraktion mit der wässrigen Lösung entfernt werden. Dieser Schritt wird auch "oxidative Entkobaltung" bezeichnet. Aus der wässrigen Kobalt(II)-salzlösung kann durch Umsetzen mit Kohlenmonoxid und Wasserstoff wieder der für die Hydroformylierung benötigte Katalysatorkomplex hergestellt werden. Der hergestellte Kobaltkatalysator wird dann aus der wässrigen Phase mit einer organischen Phase, vorzugsweise dem zu hydroformylierenden Olefin, extrahiert. Es können auch neben dem Olefin die Reaktions- und Nebenprodukte der Hydroformylierung zur Katalysatorextraktion eingesetzt werden. Die mit dem Kobaltkatalysator beladenen Olefine werden dann unter erhöhtem Druck und erhöhter Temperatur in einem Reaktor hydroformyliert.

Die DE-OS 2139630 beschreibt ein Verfahren zur Herstellung von vorwiegend geradkettigen Aldehyden durch Hydroformylierung von olefinisch ungesättigten Verbindungen, wobei in einer ersten Stufe wässrige Kobaltsalzlösungen mit Kohlenmonoxid und Wasserstoff behandelt werden, die wässrige Lösung dann in einer zweiten Stufe mit einer organischen Phase extrahiert wird und die organische Phase und ein Gemisch aus Kohlenmonoxid und Wasserstoff in eine dritte Stufe überführt werden, wo - gegebenenfalls nach Zuführung der olefinisch ungesättigten verbindungen, falls diese nicht oder nur teilweise zur Extraktion in der zweiten Stufe verwendet wurden - die Hydroformylierung erfolgt.

Die EP 0 850 905 beschreibt ein Hydroformylierungsverfahren, bei dem die Bildung des Kobaltkatalysators, die Extraktion des gebildeten Kobaltkatalysators in die organische Phase und die Hydroformylierung der Olefine in einem 1-Stufen-Prozess im Hydroformylierungsreaktor durchgeführt werden. Die nach der Entkobaltung anfallende Kobaltsalzlösung wird dabei im Kreis gefahren. In den Beispielen der EP 0 850 905 wird als wässrige Kobalt(II)-salzlösung eine wässrige Kobaltacetatlösung mit 1 Gew.-% Kobalt, gerechnet als Metall, verwendet. Es hat sich gezeigt, dass bei Verwendung derart verdünnter wässriger Kobalt(II)-salzlösungen insbesondere beim Einsatz längerkettiger Olefine nur eine geringe Beladung der zu hydroformylierenden Olefine mit aktivem Kobaltkatalysator erreicht wird, da das Volumen an wässriger Phase, das zum Einbringen höherer Kobaltmengen erforderlich ist, nur unzureichend im organischen Reaktionsmedium dispergiert werden kann. Dies äußert sich in unbefriedigenden Wertproduktausbeuten.

Beim Versuch, höher konzentrierte wässrige Kobaltsalzlösungen, z. B. Kobaltacetatlösung, einzusetzen, findet man jedoch, dass ein stabiler kontinuierlicher Dauerbetrieb über längere Zeiträume bei vollständiger Rückführung der wässrigen Kobalt(II)-salzlösung nicht störungsfrei möglich ist. So werden Ausfällungen kobalthaltiger Salze in Anlagenteilen beobachtet, die die wässrige Kobaltsalzlösung führen. Durch laufende Ergänzungen frischer Kobaltacetatlösung muss das Katalysatordefizit ausgeglichen werden. Das hat zur Folge, dass ständig ein Teil der wässrigen Kobaltsalzlösung aus dem Kreislauf genommen und in einem separaten Aufarbeitungsschritt z. B. durch Ausfällen von Kobalthydroxid und Auflösen desselben in Essigsäure aufgearbeitet und in konzentrierter Form wieder in den Prozess zurückgeführt werden muss. Dieser Umweg ist aufwendig und mit Abwasserproblemen behaftet, da Kobaltionen die Mikroorganismen in biologischen Kläranlagen schädigen.

Der vorliegenden Erfindung liegt deshalb die Aufgabe zugrunde, Olefine mit 6 bis 20 Kohlenstoffatomen unter Verwendung wässriger Kobalt(II)-salzlösungen im technischen Maßstab kontinuierlich und im stabilen Dauerbetrieb mit hoher Ausbeute zu hydroformylieren, wobei der Katalysatorkreislauf möglichst einfach, weitgehend verlustfrei und umweltfreundlich sein sollte.

Die Erfinder haben gefunden, dass unabhängig vom ursprünglich eingesetzten Kobalt(II)-salz nach längerem Dauerbetrieb das Kobalt in der wässrigen Kobalt(II)-salzlösung überwiegend als Kobalt(II)-formiat vorliegt. Dies liegt daran, dass als Nebenprodukt der kobaltkatalysierten Hydroformylierung vor allem Ameisensäureester der gegenüber den eingesetzten Olefinen um ein Kohlenstoffatom verlängerten Alkanole entstehen. Diese werden in der Reaktionszone zum Teil hydrolysiert, wobei das Formiatanion in die wässrige Kobalt(II)-salzlösung übertritt. Kobalt(II)-formiat weist jedoch bei Umgebungstemperatur nur eine Wasserlöslichkeit von etwa 1 Gew.-%, gerechnet als Kobaltmetall, auf, d. h die Wasserlöslichkeit von Kobalt(II)-formiat beträgt weniger als ein Fünftel derjenigen von Kobalt(II)-acetat. Für ein ausreichendes Katalysatorangebot sind jedoch höher konzentrierte Ausgangslösungen notwendig.

Die Erfinder haben gefunden, dass ein stabiler Dauerbetrieb bei Verwendung einer wässrigen Kobalt(II)-formiatlösung mit einer Konzentration von 1,1 bis 1,7 Gew.-%, bezogen auf Kobalt, erreicht werden kann, wenn diese ständig unter Bedingungen gehalten wird, unter denen die Löslichkeitsgrenze von Kobalt(II)-formiat in Wasser nicht überschritten wird.

Demzufolge ist Gegenstand der Erfindung ein kontinuierliches Verfahren zur Hydroformylierung von Olefinen mit 6 bis 20 Kohlenstoffatomen, bei dem
a) eine wässrige Kobalt(II)-salzlösung mit Wasserstoff und Kohlenmonoxid unter Bildung eines hydroformylierungsaktiven Kobaltkatalysators (im Folgenden auch einfach "Kobaltkatalysator") innig in Kontakt gebracht wird, die den Kobaltkatalysator enthaltende wässrige Phase in wenigstens einer Reaktionszone mit den Olefinen und gegebenenfalls einem organischen Lösungsmittel sowie Wasserstoff und Kohlenmonoxid innig in Kontakt gebracht wird, wobei der Kobaltkatalysator in die organische Phase extrahiert wird und die Olefine hydroformyliert werden,
b) der Austrag aus der Reaktionszone in Gegenwart von saurer wässriger Kobalt(II)-salzlösung mit Sauerstoff behandelt wird, wobei der Kobaltkatalysator unter Bildung von Kobalt(II)-salzen zersetzt wird und diese in die wässrige Phase zurückextrahiert werden; und die Phasen anschließend getrennt werden,
c) die wässrige Kobalt(II)-salzlösung unverändert zurück in Schritt a) geführt wird,
wobei die Kobalt(II)-salzlösung eine Konzentration von 1,1 bis 1,7 Gew.-%, bezogen auf Kobalt, aufweist und ständig unter Bedingungen gehalten wird, unter denen die Löslichkeitsgrenze von Kobalt(II)-formiat in Wasser nicht überschritten wird, indem der pH-Wert der Kobalt(II)-salzlösung im Bereich von 2 bis 4 gehalten wird und alle Anlagenteile außerhald der Reaktions Zone, die bestimmungsgemäß mit der wässrigen Kobalt(II)-salzlösung in Kontakt kommen, auf einer Temperatur von mindestens 40°C gehalten werden.

Mit anderen Worten wird die Kobalt(II)-salzlösung ständig unter Bedingungen gehalten, unter denen die Löslichkeit von Kobalt(II)-formiat in Wasser, gerechnet als Kobalt, mindestens so hoch ist, wie die Konzentration des Kobalt(II)-salzes in der eingesetzten wässrigen Kobalt(II)-salzlösung und damit hoch genug ist, dass die Lösung als Katalysatorvorstufe direkt in die Reaktionszone zurückgeführt werden kann und einen stabilen Dauerbetrieb der Oxo-Synthese ermöglicht. Vorzugsweise werden die Bedingungen so gewählt, dass die Löslichkeit mindestens 1,3 Gew.-% beträgt. Durch diese Maßnahme gelingt es, höherkonzentrierte Kobalt(II)-salzlösungen im Verfahren einzusetzen und diese ohne erneute Aufkonzentrierung und ohne Ausschleusen von Teilströmen ständig im Kreis zu fahren, ohne dass es zu verfahrensbedingten Kobaltverlusten im Katalysatorkreislauf kommt.

Im Kreislauf der wässrigen Kobalt(II)-salzlösung liegt das Kobalt im erfindungsgemäßen Verfahren im Wesentlichen als Kobalt(II)-formiat vor. Zum Anfahren der Hydroformylierungsanlage können jedoch auch z. B. Kobaltacetylacetonat sowie die Kobalt(II)-salze anderer Carbonsäuren, wie Kobaltacetat oder Kobaltethylhexanoat, verwendet werden.

Zu den Maßnahmen, die zu einer ausreichenden Löslichkeit des Kobaltformiats führen, zählt vor allem die Steuerung des pH-werts der Kobalt(II)-salzlösung und/oder der Temperatur. der Kobalt(II)-salzlösung außerhalb der Reaktionszone. So wird der pH-Wert der wässrigen Kobalt(II)-salzlösung vorzugsweise im Bereich von 2 bis 4, insbesondere 3 bis 3,5, gehalten. Das Einhalten eines pH-Werts im angegebenen Bereich verhindert, dass basische Kobalt(II)-formiate ausfallen, die nahezu wasserunlöslich sind und dadurch dem Katalysatorkreislauf verloren gehen. Zur Einhaltung des angegebenen pH-Werts ist es in der Regel erforderlich, kontinuierlich oder periodisch Säure, vorzugsweise Ameisensäure, zu der wässrigen Kobalt(II)-salzlösung zuzugeben. Der pH-Wert wird vorzugsweise kontinuierlich überwacht und die Säurezugabe bedarfsgesteuert geregelt.

Die wässrige Kobalt(II)-salzlösung außerhalb der Reaktionszone wird auf einer Temperatur von mindestens 40 °C, insbesondere 60 bis 95 °C gehalten. Dies geschieht dadurch, dass alle Anlagenteile außerhalb der Reaktionszone, die bestimmungsgemäß mit der wässrigen Kobalt(II)-salzlösung in Kontakt kommen, auf einer Temperatur von mindestens 40 °C, insbesondere 60 bis 95 °C, gehalten werden. Zu diesem Zweck ist es in der Regel erforderlich, eine ausreichende Isolierung und gegebenenfalls Beheizung der Apparate und Leitungen sowie der Mess- und Regeleinrichtungen vorzusehen.

Die Konzentration der wässrigen Kobalt(II)-salzlösung wird im Bereich von 1,1 bis 1,7 Gew.-%, insbesondere 1,3 bis 1,5 Gew.-%, bezogen auf Kobalt, gehalten. Aufgrund des Wasseraufnahmevermögens des Hydroformylierungsproduktes wird dem Kreislauf der Kobalt(II)-salzlösung kontinuierlich eine gewisse Wassermenge entzogen und bei der Phasentrennung im Schritt b) mit der organischen Phase abgetrennt (je nach Kettenlänge des zu hydroformylierenden Olefins zwischen 1 bis 3 %). Um ein Aufkonzentrieren der Kobalt(II)-salzlösung und damit ein Ausfallen von Kobalt(II)-salzen zu verhindern, wird der Wasserentzug vorzugsweise durch kontinuierliche oder periodische Wasserergänzung in den Kreislauf der Kobalt(II)-salzlösung kompensiert. Dieses gelingt auf elegante Weise durch Konstanthalten des Trennschichtniveaus im Phasentrenngefäß, in dem die von den Kobaltverbindungen befreite organische Produktphase von der wässrigen Phase getrennt wird.

Der Schritt (a) des erfindungsgemäßen Verfahrens umfasst die Stufen der Katalysatorbildung, der Katalysatorextraktion und der Hydroformylierung. Bei der Katalysatorbildung wird ausgehend von einer wässrigen Kobalt(II)-salzlösung durch Umsetzung mit Kohlenmonoxid und Wasserstoff der für die Hydroformylierung benötigte hydroformylierungsaktive Katalysatorkomplex (HCo(CO)₄) hergestellt. Bei der Katalysatorextraktion wird der hergestellte hydroformylierungsaktive Kobaltkatalysator aus der wässrigen Phase in die organische Phase extrahiert, die aus dem zu hydroformylierenden Olefin und gegebenenfalls den Reaktions- und Nebenprodukten der Hydroformylierung bzw. mitverwendeten organischen Lösungsmitteln besteht. Die Hydroformylierung erfolgt in der mit dem Kobaltkatalysator beladenen organischen Phase, wobei die Olefine in die um ein Kohlenstoffatom verlängerten Aldehyde und/oder Alkohole umgewandelt werden.

Beim erfindungsgemäßen Verfahren erfolgen die Katalysatorextraktion und Hydroformylierung in einem Schritt in der Reaktionszone des Hydroformylierungsreaktors. Die Katalysatorbildung kann gleichzeitig damit oder in einem vorgelagerten Schritt erfolgen. Die Vorab-Bildung des Katalysators außerhalb des eigentlichen Hydroformylierungsreaktors wird gelegentlich auch als vorcarbonylierung bezeichnet. Bei der Vorcarbonylierung wird die wässrige Kobalt(II)-salzlösung in Abwesenheit der zu hydroformylierenden Olefine mit Wasserstoff und Kohlenmonoxid in Kontakt gebracht, vorzugsweise bei Temperaturen von 50 bis 200 °C, insbesondere 100 bis 160 °C und unter Drucken von 100 bis 400 bar, insbesondere 200 bis 300 bar. Es eignen sich übliche Apparate für Gas-flüssig-Reaktionen, wie Rührbehälter mit Begasungsrührer, Blasensäulen oder Rieselbettkolonnen. Mit Vorteil erfolgt die Vorcarbonylierung in Gegenwart von Aktivkohle, Zeolithen oder basischen Ionenaustauschen, die mit Kobaltcarbonyl beladen sind, gemäß Beschreibung in der DE-OS 2139630. Die so erhaltene, Kobalt(II)-salze und Kobaltkatalysator enthaltende wässrige Lösung wird dann zusammen mit den zu hydroformylierenden Olefinen und gegebenenfalls mitverwendeten organischen Lösungsmitteln sowie Wasserstoff und Kohlenmonoxid in die Reaktionszone geleitet.

Die Vorcarbonylierung empfiehlt sich insbesondere bei der Hydroformylierung linearer längerkettiger Olefine mit endständiger Doppelbindung zur Herstellung von vorwiegend geradkettigen Aldehyden/Alkoholen. Um die Bildung unerwünschter verzweigter Hydroformylierungsprodukte zu minimieren, werden hierbei in der Regel niedrigere Reaktionstemperaturen angewandt. Unter diesen Bedingungen bildet sich der aktive Kobaltkatalysator nicht mit genügender Geschwindigkeit in der Reaktionszone des Hydroformylierungsreaktors.

In vielen Fällen ist es im Hinblick auf den geringeren verfahrenstechnischen Aufwand bevorzugt, dass die Bildung des Kobaltkatalysators, die Extraktion des Kobaltkatalysators in die organische Phase und die Hydroformylierung der Olefine in einem Schritt erfolgen, indem die wässrige Kobalt(II)-salzlösung, die Olefine und gegebenenfalls das organische Lösungsmittel in der Reaktionszone unter Hydroformylierungsbedingungen innig in Kontakt gebracht werden.

Die Ausgangsstoffe werden dabei so in die Reaktionszone eingebracht, dass eine gute Phasenvermischung erfolgt und eine möglichst hohe Phasenaustauschfläche erzeugt wird. Für die Dosierung können die dem Fachmann bekannten Dosiervorrichtungen, wie z. B. mit Füllkörpern befüllte Turbulenzrohre oder Mischdüsen für Mehrphasensysteme eingesetzt werden. Besonders bevorzugt erfolgt die Dosierung mit einer Mischdüse unter Aufrechterhaltung einer turbulenten Strömung in der Reaktionszone. So erfolgt in einer geeigneten Ausführungsform das innige Inkontaktbringen, indem die wässrige Kobalt(II)-salzlösung, Olefin sowie Kohlenmonoxid und Wasserstoff mittels einer Mischdüse gleichzeitig in ein Umlaufsystem, wie in den DE-AS 1205514 und 1938102 beschrieben, in die Reaktionszone eingebracht werden. Das gegebenenfalls mitverwendete organische Lösungsmittel liegt entweder in der Reaktionszone vor oder wird gleichzeitig mit den anderen Ausgangsstoffen, insbesondere mittels einer Mischdüse, in die Reaktionszone eingebracht.

Geeignete, druckfeste Reaktionsapparaturen für die Hydroformylierung sind dem Fachmann bekannt. Dazu zählen die allgemein üblichen Reaktoren für Gas-flüssig-Reaktionen, wie z. B. Rohrreaktoren, Rührkessel, Gasumlaufreaktoren, Blasensäulen usw., die gegebenenfalls durch Einbauten nochmals unterteilt sein können. Geeignet ist beispielsweise ein senkrecht stehender Hochdruck-Blasensäulenreaktor, der gegebenenfalls mit koaxialen rohrförmigen Einbauten versehen ist.

Kohlenmonoxid und Wasserstoff werden üblicherweise in Form eines Gemisches, dem sogenannten Synthesegas, eingesetzt. Die Zusammensetzung des im erfindungsgemäßen Verfahren eingesetzten Synthesegases kann in weiten Bereichen variieren. Das molare Verhältnis von Kohlenmonoxid und Wasserstoff beträgt in der Regel etwa 10:1 bis 1:10, insbesondere 2,5:1 bis 1:2,5. Ein bevorzugtes Verhältnis liegt bei etwa 1:1,5.

Als gegebenenfalls mitzuverwendende organische Lösungsmittel kommen inerte Kohlenwasserstoffe, wie Paraffinfraktionen, aromatische Kohlenwasserstoffe, wie Benzol, Toluol oder Xylol, oder ein Aldehyd und/oder Alkohol, insbesondere aber das Hydroformylierungsprodukt des eingesetzten Olefins, in Betracht. Es können ferner hochsiedende Nebenprodukte der Hydroformylierung als Lösungsmittel verwendet werden. Die Mitverwendung eines Lösungsmittels kann z. B. zur Viskositätserniedrigung bei langkettigen Olefinen vorteilhaft sein.

Die Temperatur bei der Hydroformylierung liegt im Allgemeinen bei 100 bis 250 °C, insbesondere 145 bis 200 °C. Die Umsetzung wird vorzugsweise bei einem Druck im Bereich von 100 bis 400 bar, insbesondere 200 bis 300 bar, durchgeführt.

Bei Verwendung eines vertikalen Rohrreaktors, der wie ausgeführt Einbauten aufweisen kann, wird der Reaktionsaustrag üblicherweise am Kopf des Reaktors entnommen. Insbesondere bei höheren Olefinen mit 9 Kohlenstoffatomen oder mehr wird die der Reaktionszone zugeführte wässrige Phase, die zur Erreichung einer ausreichenden Katalysatorkonzentration in der Reaktionszone notwendig ist, unter Umständen nicht vollständig in gelöster oder suspendierter Form mit dem über Kopf abgenommenen Reaktionsgemisch abgeführt. Dies kann zur Ansammlung wässriger Phase in der Reaktionszone und zum allmählichen Rückgang und gegebenenfalls zum gänzlichen Erliegen der Hydroformylierungsreaktion führen. Daher wird in einer bevorzugten Ausführungsform Reaktionsaustrag sowohl am Kopf des Reaktors als auch aus dem Sumpfraum des Reaktors entnommen. Der Reaktionsaustrag aus dem Sumpfraum umfasst vorzugsweise 10 bis 80 vol.-%, insbesondere 30 bis 50 Vol.-%, wässrige Phase. Die Entnahme des Reaktionsaustrags aus dem Sumpfraum erfolgt vorzugsweise phasengeregelt. Der Sumpfabzug beträgt, abhängig von der Kobaltkonzentration der wässrigen Kobalt(II)-salzlösung und der Kettenlänge des zu hydroformylierenden Olefins, in der Regel etwa 10 bis 40 Gew.-%, bezogen auf das eingesetzte Olefin.

Der Sumpfabzug aus der Hydroformylierungszone enthält neben wässriger Phase wesentliche Mengen an teilumgesetzter organischer Phase. Es ist daher im Sinne einer möglichst hohen Wertproduktausbeute bevorzugt, die Hydroformylierung in einer Reaktorkaskade durchzuführen. In einer bevorzugten Ausführungsform werden daher der am Kopf des Reaktors und der aus dem Sumpfraum entnommene Reaktionsaustrag gemischt und in einer zweiten Reaktionszone erneut Hydroformylierungsbedingungen unterworfen. Alternativ kann nur der Reaktionsaustrag aus dem Sumpfraum der zweiten Reaktionszone zugeführt werden. In die zweite Reaktionszone kann gegebenenfalls frisches Synthesegas eingebracht werden. Ein gleichmäßiger Stofftransport von der ersten Reaktionszone zur zweiten Reaktionszone erfolgt vorzugsweise durch Aufrechterhalten eines konstanten Differenzdrucks von wenigen bar, z. B. 2 bis 5 bar.

Der Reaktionsaustrag, der wie erwähnt, in einer zweiten Reaktionszone erneut Hydroformylierungsbedingungen unterworfen sein kann, wird geeigneterweise nach Verlassen der Reaktionszone auf Mitteldruck, in der Regel 10 bis 30 bar, entspannt und in die Entkobaltungsstufe geleitet. In der Entkobaltungsstufe wird der Reaktionsaustrag in Gegenwart von wässriger, schwach saurer Kobalt(II)-salzlösung mit Luft oder Sauerstoff bei Temperaturen von vorzugsweise 90 bis 130 °C von Kobaltcarbonylkomplexen befreit. Die Entkobaltung kann gewünschtenfalls in einem mit Füllkörpern, wie z. B. Raschig-Ringen, befüllten Druckbehälter durchgeführt werden, in dem eine möglichst hohe Phasenaustauschfläche erzeugt wird. In einem nachgeschalteten Phasentrenngefäß wird die organische Produktphase von der wässrigen Phase getrennt. Bei der Entkobaltung wird der hydroformylierungsaktive Kobaltkatalysator unter Bildung von Kobalt(II)-salzen, überwiegend Kobalt(II)-formiat, zersetzt. Die Kobalt(II)-salze werden in die wässrige Phase zurückextrahiert. Gleichzeitig geht die Kobalt-abgereicherte wässrige Phase, die nach der Katalysatorextraktion zurückbleibt und während der Hydroformylierung in der Reaktionszone vorliegt und mit dem Reaktionsaustrag in die Entkobaltungsstufe gelangt, in die wässrige Kobalt(II)-salzlösung über. Die Konzentrationserhöhung durch die Rückextraktion der Kobalt(II)-salze und die Konzentrationserniedrigung durch Verdünnung mit der Kobalt-abgereicherten wässrigen Phase heben sich im Wesentlichen gegenseitig auf, so dass nach der Entkobaltung eine Kobalt(II)-salzlösung mit im Wesentlichen der ursprünglichen Konzentration erhalten wird. Erfindungsgemäß wird die wässrige Kobalt(II)-salzlösung unverändert, d. h. ohne chemische Aufarbeitung oder Aufkonzentrieren, in die Reaktionszone bzw. Katalysatorbildungsstufe zurückgeführt.

Anschließend kann die nach Abtrennung der wässrigen Phase verbleibende organische Phase geeignet aufgearbeitet, z. B. destilliert und/oder hydriert, werden.

Nach dem erfindungsgemäßen Verfahren lassen sich Olefine mit 6 bis 20 Kohlenstoffatomen hydroformylieren. Das erfindungsgemäße Verfahren ist insbesondere für die Hydroformylierung von isomeren olefingemischen geeignet, die durch Oligomerisierung von niederen Olefinen, wie Propen und Butenen, hergestellt werden. Zu den typischen Oligomerisaten, die als Ausgangsprodukte für das vorliegende Verfahren geeignet sind, zählen unter anderem Di-, Tri- und Tetrapropen, Di-, Tri- und Tetrabuten sowie Mischoligomerisate von Propenen und Butenen. Die Oligomerisate von Butenen sind nach bekannten Oligomerisierungsverfahren, z. B. nach dem Octol®-Prozess von Hüls und dem Dimersol®-Verfahren von IFP, großtechnisch zugänglich. Nach dem erfindungsgemäßen Verfahren können ferner lineare langkettige Olefine mit endständiger Doppelbindung, die z. B. nach dem SHOP®-Prozess oder Ziegler-Verfahren erhältlich sind, oder lineare langkettige Olefine mit innenliegender Doppelbindung hydroformyliert werden.

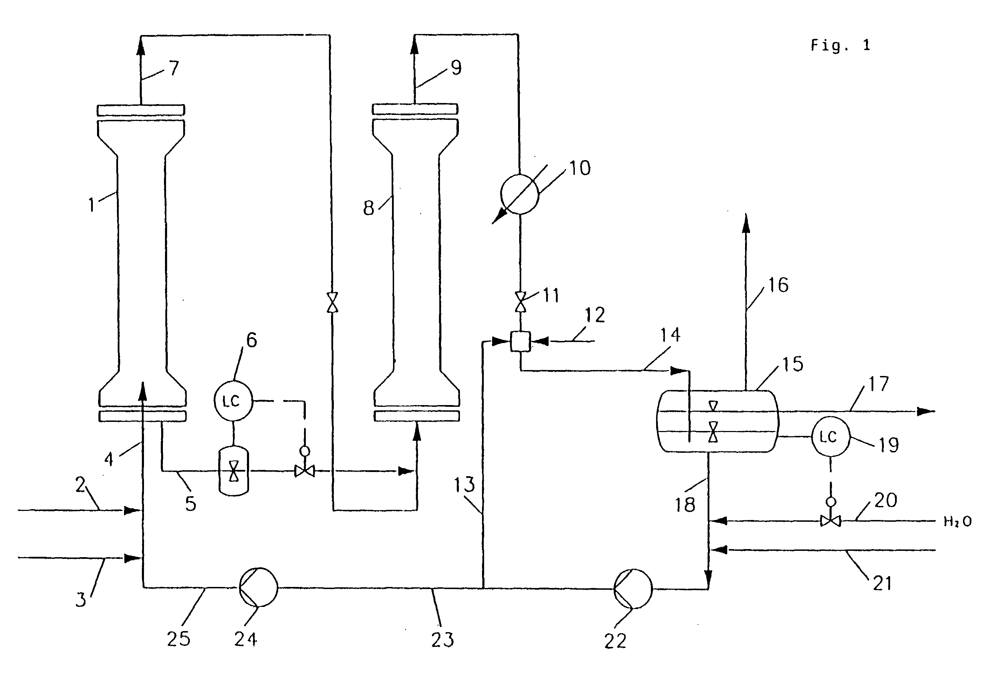

Anhand der beigefügten Fig. 1 wird nun eine bevorzugte Ausführungsform der Erfindung näher erläutert. Fig. 1 ist die schematische Darstellung einer zur Durchführung des erfindungsgemäßen Verfahrens geeigneten Anlage. An sich selbstverständliche Anlagendetails, die für das Verständnis der Erfindung nicht erforderlich sind, wurden der Übersichtlichkeit halber weggelassen.

Einem senkrecht stehenden Hochdruckreaktor 1, der mit Einrichtungen zur intensiven Durchmischung der Reaktionspartner sowie zur Abführung der Reaktionswärme ausgerüstet ist, werden über die Leitungen 2, 3 und 25 Olefine (≥ C₆), Oxogas und wässrige Kobaltformiatlösung mit einem Co-Gehalt von 1,1 bis 1,7 Gew.-% zugeführt.

Der über Kopf abgezogene Reaktoraustrag gelangt über Leitung 7 zum Nachreaktor 8, der ebenfalls mit Einrichtungen zur Wärmeabfuhr ausgestattet ist. Zwischen Haupt- und Nachreaktor wird ein Druckgefälle von 2 bis 5 bar eingestellt. Am Boden des Reaktors 1 zieht man über Leitung 5 einen Teil des Reaktorinhalts ab und vereinigt ihn mit dem Zulauf zum Nachreaktor 8. Dieser Sumpfabzug besteht, abhängig vom eingesetzten Olefin und von der Konzentration der Kobaltformiatlösung, zu 10 bis 80 % aus wässriger Phase, wobei der Rest organische Phase ist. Für die Einhaltung eines bestimmten Phasenverhältnisses (z. B. 50:50) sorgt die Phasenregelung 6.

Über Leitung 9 verlässt das Reaktionsgemisch den Nachreaktor 8, passiert den Kühler 10 und wird im Entspannungsventil 11 vom Reaktordruck (> 200 bar) auf Mitteldruck (10 bis 30 bar) entspannt. Unmittelbar nach dem Drucksprung wird über die Leitung 12 Luft und über Leitung 13 ameisensaure Kobaltformiatlösung (Kobaltgehalt 1,1 bis 1,7 Gew.-%) dem Reaktoraustrag zugeführt.

Über Leitung 14 gelangt das dreiphasige Gemisch zum Trennbehälter 15. Das freiwerdende überschüssige Synthesegas verlässt die Anlage über Leitung 16. Die organische, im wesentlichen kobaltfreie Oberphase wird zur Weiterverarbeitung über Leitung 17, die Wasserphase über Leitung 18 abgezogen. Die Trennschichtregelung 19 ergänzt über Leitung 20 ständig so viel Wasser wie das organische Reaktionsprodukt aufgrund seines Wasseraufnahmevermögens austrägt.

Mit der Pumpe 22 wird die wässrige Kobaltformiatlösung (Kobaltgehalt 1,1 bis 1,7 Gew.-%) über Leitung 13 der Entkobaltungsstufe und über Leitung 23 der Hochdruckpumpe 24 zugeführt. Schließlich wird über Leitung 25 der Reaktorzulauf mit der erforderlichen Menge (etwa 0,13 Gew.-%, bezogen auf Olefin) der 1,1 bis 1,7 prozentualen Kobaltformiatlösung versetzt.

Der Kobaltkreislauf ist in sich vollständig geschlossen. Er ist autark. Es besteht keine Notwendigkeit, einen Teilstrom abzuzweigen und einer separaten Aufarbeitung bzw. Aufkonzentrierung zuzuführen. Die mit dem Hydroformylierungsprodukt ausgetragenen minimalen Verluste von höchstens etwa 3 ppm Kobalt (bezogen auf das organische Reaktionsprodukt) müssen durch gelegentliche Ergänzung von konzentrierter Kobalt(II)-salzlösung, z. B. 6-prozentige Kobaltacetatlösung, über Leitung 21 ausgeglichen werden, ebenso der Schwund durch Leckagen, z. B. an Pumpen.

Der pH-Wert der wässrigen Unterphase im Trenngefäß 15 unterliegt einer regelmäßigen Kontrolle und wird durch bedarfsweise Ameisensäureergänzungen über die Leitung 21 im Bereich zwischen 2 und 4 gehalten.

Die Leitungen 18, 13, 23 und 25 sowie die Pumpen 22 und 24 sind mit einer Begleitheizung, z. B. in Form elektrischer Heizbänder oder mit Heizmedium durchströmter Rohre, versehen.

### Beispiel 1

Das erfindungsgemäße Verfahren wird in einer technischen Anlage, die unter Bezugnahme auf Fig. 1 aufgebaut ist, durchgeführt.

Der Reaktor 1, der mit Einrichtungen zur Wärmeabfuhr und einer Mischdüse zur Vermischung und zum Eintrag der Reaktionspartner in ein Umlaufsystem ausgerüstet ist, verfügt über einen nutzbaren Reaktionsraum von 11,5 m³, der entsprechende Reaktionsraum des Nachreaktors 8 liegt bei 8,5 m³. Die Reaktionsbedingungen für den ersten Reaktor betragen 187 ° ± 1 °C und 275 bar. Die Temperatur im Nachreaktor liegt auf gleichem Niveau, der Druck wird 3 bar unter dem Druck des ersten Reaktors gehalten.

Dem zum Start mit Rohnonanol befüllten Reaktor 1 werden stündlich 4.500 kg eines Oktenisomerengemisches aus der Butendimerisierung nach dem IFP-Verfahren zusammen mit 420 kg einer 1,4 Gew.-% Kobalt enthaltenden wässrigen Kobaltformiatlösung und druckgeregelt 1.460 kg einer Synthesegasmischung (39,5 Mol.-% CO-Gehalt) zugeführt.

Der Hauptteil des Reaktionsgemisches verlässt den Reaktor 1 über Kopf und passiert anschließend den Nachreaktor 8. Dem Reaktorsumpf werden kontinuierlich etwa 500 kg/Std. Reaktorinhalt über die Phasenregelung 6 entnommen und dem Zulauf zum Nachreaktor beigemischt. Dieser Sumpfabzug besteht im Mittel zu 1/3 aus wässriger Phase. Der Austrag des Nachreaktors wird im Wärmetauscher 10 auf unter 150 °C gekühlt und im Entspannungsventil 11 auf etwa 20 bar entspannt.

Unmittelbar nach dem Drucksprung werden dem Reaktionsprodukt etwa 4.000 kg/Std. Kobaltformiatlösung mit einem mittleren pH-Wert von 3 und 21 kg/Std. Luft beigemischt. Aus dem Phasentrennbehälter 15 zieht man das freiwerdende Entspannungsgas ab. Die aufschwimmende organische Oberphase mit einem Restkobaltgehalt von 1 bis 2 ppm wird kontinuierlich ausgetragen und liefert nach Hydrierung und Destillation 4.940 kg eines reinen i-Nonylalkoholgemisches, entsprechend 85,4 % der Theorie.

Die Oberphase entzieht aufgrund ihres Wasseraufnahmevermögens dem wässrigen Katalysatorsystem etwa 2 Gew.-% Wasser. Diese Menge muss über die Trennschichtregelung 19 ständig ergänzt werden, um die Kobaltkonzentration bei 1,4 % zu halten. Die wässrige Unterphase aus dem Trennbehälter 15 versorgt über die Pumpe 22 sowohl den Reaktoraustrag nach der Entspannung mit etwa 4.000 kg/Std. wässriger Formiatlösung als auch die Katalysatorpumpe 24 mit 420 kg Formiatlösung, um daraus im Reaktor in situ Kobaltcarbonyle zu bilden.

Im Abstand von 1 bis 2 Wochen werden die mit der organischen Phase ausgetragenen Kobaltmengen sowie Leckageverluste durch Zufuhr von 6 % Co enthaltender wässriger Kobaltacetatlösung über Leitung 21 ausgeglichen. Fallweise werden über diese Leitung auch die Ameisensäureergänzungen vorgenommen, um den pH-Wert der Formiatlösung in dem angegebenen Bereich von 2 bis 4 zu halten (im Durchschnitt etwa 5 kg/d 100-prozentige Ameisensäure).

### Vergleichsbeispiel 1

Man benutzt dieselbe Anlage wie in Beispiel 1 und arbeitet mit einer Formiatlösung, die statt 1,4 % nur 1 % Kobalt enthält.

Bei gleicher Olefinzufuhr wie im Beispiel 1 muss die dem Reaktor zugepumpte Formiatlösung auf 590 kg erhöht werden. Die Sumpfabzugsmenge aus Reaktor 1 verdoppelt sich bei in etwa gleichem Verhältnis von organischer zu wässriger Phase (2:1). Die übrigen Anlagedaten bleiben im Wesentlichen unverändert. Die Reinalkoholausbeute sinkt auf 4.700 kg entsprechend 81,4 % der Theorie.

### Vergleichsbeispiel 2

Es werden die Einstellungen des Vergleichsbeispiels 1 übernommen; zusätzlich wird der Sumpfabzug aus Reaktor 1 geschlossen.

Bereits nach wenigen Stunden ist ein deutlich rückläufiger Synthesegasverbrauch festzustellen. Um die Reaktion einigermaßen aufrecht zu erhalten, muss die Umsetzungstemperatur auf > 190 °C erhöht werden. Der Reaktoraustrag enthält noch beträchtliche Anteile an unumgesetztem bzw. hydriertem Einsatzolefin. Die Reinalkoholausbeute sinkt auf etwa 2.500 kg, entsprechend 43,2 % der Theorie.

### Beispiel 2

Das erfindungsgemäße Verfahren wird in der im Beispiel 1 beschriebenen technischen Anlage durchgeführt.

Die Reaktionsbedingungen für den ersten Reaktor betragen 192 ± 1 °C und 275 bar. Die Temperatur im Nachreaktor liegt auf gleichem Niveau, der Druck wird 3 bar unter dem des ersten Reaktors gehalten.

Dem zum Start mit Rohtridekanol befüllten Reaktor 1 werden stündlich 3.500 kg eines Dodecenisomerengemisches aus der Butentrimerisierung nach dem IFP-Verfahren zusammen mit 310 kg einer 1,5 Gew.-% Kobalt enthaltenden wässrigen Kobaltformiatlösung und druckgeregelt 790 kg einer Synthesegasmischung (39,5 Mol.-% CO-Gehalt) zugeführt.

Man verfährt wie in Beispiel 1 und entnimmt phasengeregelt dem Sumpf von Reaktor 1 etwa 500 kg Reaktorinhalt, der zu 1/3 aus Wasser besteht. Im Übrigen verfährt man analog zu Beispiel 1.

Nach Hydrierung und Destillation liefert die organische Oberphase 3.350 kg eines reinen Tridekanolisomerengemisches, entsprechend 80,4 % der Theorie.

Auch hier, wie bereits in Beispiel 1 beschrieben, sorgt die Trennschichtregelung 19 für den Trennbehälter 15 automatisch für die Beibehaltung der Kobaltkonzentration in der Formiatlösung, in diesem Falle 1,5 %, wobei aufgrund des verringerten Wasseraufnahmevermögens des C₁₃-Hydroformylierungsproduktes etwas weniger Wasser ergänzt werden muss.

## Patentansprüche

1. Kontinuierliches Verfahren zur Hydroformylierung von Olefinen mit 6 bis 20 Kohlenstoffatomen, bei dem
a) eine wässrige Kobalt(II)-sal-zlösung mit Wasserstoff und Kohlenmonoxid unter Bildung eines hydroformylierungsaktiven Kobaltkatalysators innig in Kontakt gebracht wird, die den Kobaltkatalysator enthaltende wässrige Phase in wenigstens einer Reaktionszone mit den Olefinen und gegebenenfalls einem organischen Lösungsmittel sowie Wasserstoff und Kohlenmonoxid innig in Kontakt gebracht wird, wobei der Kobaltkatalysator in die organische Phase extrahiert wird und die Olefine hydroformyliert werden,
b) der Austrag aus der Reaktionszone in Gegenwart von saurer wässriger Kobalt(II)-salzlösung mit Sauerstoff behandelt wird, wobei der Kobaltkatalysator unter Bildung von Kobalt(II)-salzen zersetzt wird und diese in die wässrige Phase zurückextrahiert werden; und die Phasen anschließend getrennt werden,
c) die wässrige Kobalt(II)-Salzlösung unverändert zurück in Schritt a) geführt wird,
wobei die Kobalt(II)-salzlösung eine Konzentration von 1,1 bis 1,7 Gew.-%, bezogen auf Kobalt, aufweist und ständig unter Bedingungen gehalten wird, unter denen die Löslichkeitsgrenze von Kobalt(II)-formiat in Wasser nicht überschritten wird, indem der pH-Wert der Kobalt(II)-salzlösung im Bereich von 2 bis 4 gehalten wird und alle Anlagenteile außerhalb der Reaktionszone, die bestimmungsgemäß mit der wässrigen Kobalt(II)-salzlösung in Kontakt kommen, auf einer Temperatur von mindestens 40 °C gehalten werden.

2. Verfahren nach Anspruch 1, **dadurch gekennzeichnet, dass** der pH-Wert durch Zugabe von Ameisensäure eingestellt wird.

3. Verfahren nach einem der vorherigen Ansprüche, **dadurch gekennzeichnet, dass** die Wassermenge, die sich in der organischen Phase löst und der wässrigen Kobalt(II)-salzlösung entzogen wird, kontinuierlich oder periodisch ergänzt wird.

4. Verfahren nach einem der vorhergehenden Ansprüche, **dadurch gekennzeichnet, dass** die Bildung des Kobaltkatalysators, die Extraktion des Kobaltkatalysators in die organische Phase ur die Hydroformylierung der Olefine in einem Schritt erfolgen, indem die wässrige Kobalt(II)-salzlösung, die Olefine und gegebenenfalls das organische Lösungsmittel sowie Wasserstoff und Kohlenmonoxid in der Reaktionszone unter Hydroformylierungsbedingungen innig in Kontakt gebracht werden.

5. Verfahren nach Anspruch 4, **dadurch gekennzeichnet, dass** das Inkontaktbringen erfolgt, indem die wässrige Kobalt(II)-salzlösung, die Olefine und gegebenenfalls das organische Lösungsmittel sowie Kohlenmonoxid und Wasserstoff mittels einer Mischdüse in die Reaktionszone eingebracht werden.

6. Verfahren nach einem der vorhergehenden Ansprüche, **dadurch gekennzeichnet, dass** die Reaktionszone ein vertikaler Rohrreaktor ist, wobei Reaktionsaustrag sowohl am Kopf des Reaktors als auch aus dem Sumpfraum des Reaktors entnommen wird.

7. Verfahren nach Anspruch 6, **dadurch gekennzeichnet, dass** der Reaktionsaustrag aus dem Sumpfraum 10 bis 80 Vol.-% wässrige Phase umfasst.

8. Verfahren nach Anspruch 6 oder 7, **dadurch gekennzeichnet, dass** der am Kopf des Reaktors und der aus dem Sumpfraum des Reaktors entnommene Reaktionsaustrag gemischt und in einer zweiten Reaktionszone erneut Hydroformylierungsbedingungen unterworfen werden.

9. Verfahren nach einem der vorhergehenden Ansprüche, **dadurch gekennzeichnet, dass** das Kobalt in der wässrigen Kobalt(II)-salzlösung im Wesentlichen als Kobalt(II)-formiat vorliegt.

## Claims

1. A continuous process for the hydroformylation of olefins having from 6 to 20 carbon atoms, in which
a) an aqueous cabalt(II) salt solution is brought into intimate contact with hydrogen and carbon monoxide to form a hydroformylation-active cobalt catalyst, and the aqueous phase comprising the cobalt catalyst is brought into intimate contact with the olefins and, if desired, an organic solvent and also hydrogen and carbon monoxide in at least one reaction zone where the cobalt catalyst is extracted into the organic phase and the olefins are hydroformylated,
b) the output from the reaction zone is treated with oxygen in the presence of acidic aqueous cobalt(II) salt solution, with the cobalt catalyst being decomposed to form cobalt(II) salts and these being backextracted into the aqueous phase; and the phases are subsequently separated,
c) the aqueous cobalt(II) salt solution is recirculated in unchanged form to step a),
wherein the cobalt(II) salt solution has a concentration of from 1.1 to 1.7% by weight, calculated as cobalt, and is continually maintained under conditions under which the solubility limit of cobalt(II) formate in water is not exceeded, wherein the pH of the cobalt(II) salt solution is maintained in the range from 2 to 4, and
wherein all plant components outside the reaction zone which in normal operation come into contact with the aqueous cobalt(II) salt solution are maintained at a temperature of at least 40°C.

2. A process as claimed in claim 1, wherein the pH is adjusted by addition of formic acid.

3. A process as claimed in any of the preceding claims, wherein the water which dissolves in the organic phase and is removed from the aqueous cobalt(II) salt solution is replaced continuously or periodically.

4. A process as claimed in any of the preceding claims, wherein the formation of the cobalt catalyst, the extraction of the cobalt catalyst into the organic phase and the hydroformylation of the olefins are carried out in one step by bringing the aqueous cobalt(II) salt solution, the olefins and any organic solvent and also hydrogen and carbon monoxide into intimate contact with one another in the reaction zone under hydroformylation conditions.

5. A process as claimed in claim 4, wherein the bringing into intimate contact is achieved by introducing the aqueous cobalt(II) salt solution, the olefins and any organic solvent and also carbon monoxide and hydrogen into the reaction zone by means of a mixing nozzle.

6. A process as claimed in any of the preceding claims, wherein the reaction zone is a vertical tube reactor and the reaction product is taken off both at the top of the reactor and also from the bottom zone of the reactor.

7. A process as claimed in claim 6, wherein the reaction product taken from the bottom zone comprises from 10 to 80% by volume of aqueous phase.

8. A process as claimed in claim 6 or 7, wherein the reaction product taken off at the top of the reactor and that taken off from the bottom zone of the reactor are mixed and subjected once again to hydroformylation conditions in a second reaction zone.

9. A process as claimed in any of the preceding claims, wherein the cobalt in the aqueous cobalt(II) salt solution is present essentially as cobalt(II) formate.

## Revendications

1. Procédé continu d'hydroformylation d'oléfines ayant 6 à 20 atomes de carbone, dans lequel
a) une solution saline aqueuse de cobalt(II) est mise en contact intime avec de l'hydrogène et du monoxyde de carbone par formation d'un catalyseur du cobalt actif pour l'hydroformylation, la phase aqueuse contenant le catalyseur du cobalt est mise en contact intime dans au moins une zone de réaction avec les oléfines et le cas échéant un solvant organique ainsi que l'hydrogène et le monoxyde de carbone, le catalyseur du cobalt étant extrait dans la phase organique et les oléfines étant hydroformylées,
b) l'extrait de la zone de réaction est traité en présence de solution saline aqueuse acide de cobalt(II) avec de l'oxygène, le catalyseur du cobalt étant détruit par formation de sels de cobalt(II) et ceux-ci étant extraits de nouveau dans la phase aqueuse ; et les phases sont ensuite séparées,
c) la solution saline aqueuse de cobalt(II) est reconduite de nouveau sans modification dans l'étape a), la solution saline de cobalt(II) présentant une concentration de 1,1 à 1,7 % en poids par rapport au cobalt et étant maintenue constamment dans des conditions dans lesquelles la limite de solubilité du formiate de cobalt(II) dans l'eau n'est pas dépassée, en ce que la valeur du pH de la solution saline de cobalt(II) est maintenue dans le domaine de 2 à 4 et toutes les parties d'installation en dehors de la zone de réaction qui entrent en contact avec la solution aqueuse de cobalt(II), selon les dispositions, sont maintenues à une température d'au moins 40°C.

2. Procédé selon la revendication 1, **caractérisé en ce que** la valeur du pH est mise au point par ajout d'acide formique.

3. Procédé selon l'une des revendications précédentes, **caractérisé en ce que** la quantité d'eau qui se dissout dans la phase organique et est enlevée à la solution saline aqueuse de cobalt(II) est complétée continuellement ou périodiquement.

4. Procédé selon l'une des revendications précédentes, **caractérisé en ce que** la formation du catalyseur du cobalt, l'extraction du catalyseur du cobalt dans la phase organique et l'hydroformylation des oléfines s'accomplit en une étape, dans laquelle la solution saline aqueuse de cobalt(II), les oléfines et le cas échéant le solvant organique ainsi que l'hydrogène et le monoxyde de carbone sont mis en contact intime dans la zone de réaction sous des conditions d'hydroformylation.

5. Procédé selon la revendication 4, **caractérisé en ce que** la mise en contact s'accomplit **en ce que** la solution saline aqueuse de cobalt(II), les oléfines et le cas échéant le solvant organique ainsi que le monoxyde de carbone et l'hydrogène sont amenés dans la zone de réaction à l'aide d'une tuyère de mélange.

6. Procédé selon l'une des revendications précédentes, **caractérisé en ce que** la zone de réaction est un réacteur tubulaire vertical, l'extrait de réaction étant prélevé aussi bien à la tête du réacteur que dans l'espace de bas de colonne du réacteur.

7. Procédé selon la revendication 6, **caractérisé en ce que** l'extrait de réaction de l'espace de bas de colonne comprend 10 à 80 % en volume de phase aqueuse.

8. Procédé selon la revendication 6 ou 7, **caractérisé en ce que** l'extrait de réaction prélevé à la tête du réacteur et celui prélevé dans l'espace de bas de colonne du réacteur sont mélangés et soumis de nouveau à des conditions d'hydroformylation dans une seconde zone de réaction.

9. Procédé selon l'une des revendications précédentes, **caractérisé en ce que** le cobalt se trouve principalement sous forme de formiate de cobalt(II) dans la solution saline aqueuse de cobalt(II).
